# EUROPEAN PATENT SPECIFICATION

(11) **EP 3 030 103 B1**
(45) Date of publication and mention of the grant of the patent: **04.12.2019**
(21) Application number: 14747392.0
(22) Date of filing: 05.08.2014
(51) Int. Cl.: A43B 5/14, A43B 7/20, A61F 5/01

(54) **SPORTS AND/OR ORTHOPAEDIC FOOTWEAR WITH DEVICE FOR RELATIVE ARTICULATION BETWEEN INSOLE AND UPPER**
SPORT- UND/ODER ORTHOPÄDISCHER SCHUH MIT VORRICHTUNG ZUR RELATIVEN BEWEGUNG ZWISCHEN INNENSOHLE UND SCHAFT MITTELS GELENK
CHAUSSURE DE SPORT ET/OU ORTHOPEDIQUE AVEC UN DISPOSITIF POUR L'ARTICULATION RELATIVE ENTRE LA SEMELLE INTERIEURE ET LA TIGE

(30) Priority: 08.08.2013 IT MI20131363
(43) Date of publication of application: 15.06.2016
(73) Proprietor: Barozzi, Alberto, 22030 Caslino d'Erba (CO) (IT)
(72) Inventor: Barozzi, Alberto, 22030 Caslino d'Erba (CO) (IT)
(74) Representative: Raimondi, Margherita
(86) International application number: PCT/EP2014/066811
(87) International publication number: WO 2015/018826

(56) References cited:
- US-A- 5 611 773
- US-A- 5 865 778
- US-A- 6 155 998
- US-A1- 2009 216 164
- US-A1- 2011 067 271

## Description

The present invention relates to sports and/or orthopaedic footwear with a device for relative articulation of insole/upper.

It is known that, in numerous sporting activity sectors such as motorcycle racing, winter disciplines (skiing, snowboarding, snowmobile racing) and/or mountain climbing, the foot is subject to relative movements with respect to the leg such as rotations about the longitudinal axis of the foot, known as supination and pronation, and/or rotation about an axis perpendicular to the longitudinal axis, known as plantar flexion and dorsal extension; said relative movements are made possible by the ankle articulation.

It is also known that, during the sporting activity, the plantar rotations (forwards rotation) and even more so dorsal rotations (backwards rotation) of the foot constitute one of the main causes of injuries in the tibiotarsal region of the person practicing the activity.

In order to limit these injuries, motorcyclist boots have been proposed, as for example known from EP 0,950,361, these boots comprising means keeping the foot and the leg sections hinged together by means of an articulated joint which is positioned on the centre of the malleoli and the stiffness of which can be varied by means of an adjustment of the relative friction between the rotating parts. Although performing their function, these boots, however, have the drawback that they do not allow effective locking of the rotational movement of the foot, said rotation being controlled only by the friction which is adjusted by means of screws; this results in a variation, over time, of the adjustment due to slackening of the screws, as a result of vibration and/or wear of the friction material, which arise during use, with consequent loss of the adjustment position and a return into a condition where there is a risk of injury.

Similar prior art examples are illustrated in US 5,865,778 and US 5,611,773 which describe an articulation device for guards comprising two arms hinged together on a pin centred on the malleolus of the user and respectively connected to the insole and the upper of the footwear.

The technical problem which is posed, therefore, is that of providing sports and/or orthopaedic footwear provided with means which, once they have been preset for the specific use of the footwear, are able to limit effectively and irreversibly all the relative movements of the foot with respect to the ankle, including pronation and supination.

In particular, a motorcyclist's boot provided with means able to limit the plantar and dorsal rotation of the foot with respect to the leg is required.

In connection with this problem, it is also required that the footwear should be easy and inexpensive to produce and assemble, be able to be easily worn by the user and at the same time should have a high degree of fitting comfort.

These results are obtained according to the present invention by sports and/or orthopaedic footwear with a device for relative articulation of the upper and insole according to Claim 1. Preferred embodiments are specified in the dependent claims.

Further details may be obtained from the following description of a non-limiting example of embodiment of the subject of the present invention, provided with reference to the accompanying drawings, in which:
Figure 1: shows an exploded perspective view of the insole with sole reinforcement and part of the anti-rotational means of the footwear according to the present invention;
Figure 2: shows a front, perspective, diagrammatic view of the footwear according to Fig. 1 with the anti-rotational means positioned on both sides of the foot (not shown);

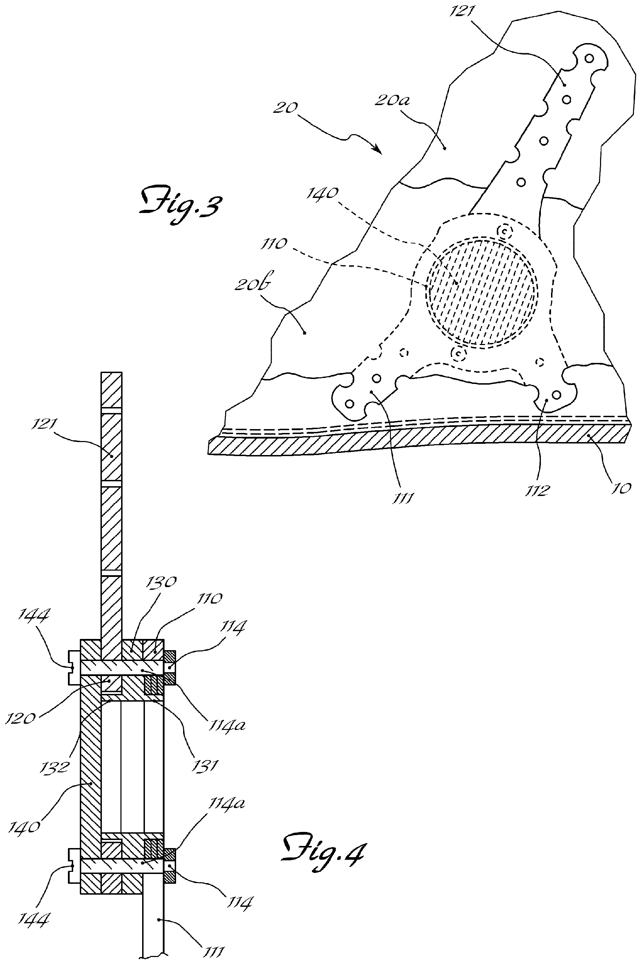

Figure 3: shows a diagrammatic cross-sectional view along the plane indicated by III-III in Fig. 2;
Figure 4: shows a schematic cross-section along the plane indicated by IV-IV in Fig. 3;
Figure 5: shows views of the detail of the rotation adjustment means in two different end-of-travel positions;
Figure 6: shows a rear view of a preferred embodiment of an adjustable upper for the footwear according to the invention;
Figure 7: shows a side view of the upper according to Fig. 6;
Figure 8: shows a rear view of the upper according to Fig. 6 in the assembled condition;
Figure 9: shows a perspective view of the upper according to Fig. 8;
Figure 10: shows a rear view and bottom view, respectively, of a preferred asymmetrical embodiment of the sole of the footwear according to the invention;
Figure 11: shows a front view, side views and a cross-sectional view of the detail of a preferred embodiment of the reinforced toe of the footwear according to the invention; and
Figure 12: shows a facing-end view, rear perspective view and cross-sectional view of the detail of a preferred embodiment of the heel with reinforcing element.

As shown and assuming for the sake of easier description and without a limiting meaning a set of three reference axes extending in a longitudinal direction X-X parallel to the length of the sole, transverse direction Y-Y parallel to the width of the sole and vertical direction Z-Z, perpendicular to the preceding directions, as well as a front part corresponding to the toe of the footwear, a rear part corresponding to the heel thereof, an inner side and an outer side respectively corresponding to the inner flank and outer flank of the user's foot, the footwear according to the invention comprises substantially:
- an upper 20 formed by a top-upper part 20a and a bottom-upper part 20b;
- an insole 10 with an inner side 10a and outer side 10b, which is fixed to the upper 20 by means of stitching, gluing or equivalent technology suitable for fastening the insole to the bottom upper 20b;

- a controlled-articulation device 100 applied to the upper 20 and comprising from the inside towards the outside:
- a first ring 110 which has, extending therefrom, a bottom-upper 20b front arm 111 and a rear bottom-upper 20b arm 112;
said first ring 110 has two radial, inner, diametrically opposite seats 113 for receiving a respective pin 114 with female thread 114a. Preferably, as can be seen from the example shown, one of the two seats is arranged between the two said bottom-upper 20b arms 111,112.

Once the pins have been inserted inside the respective seat, they are generally locked in position in a conventional manner.

Each bottom-upper arm 111,112 also has a plurality of respective holes 111c,112c which are uniformly spaced along its length so as to allow fixing of the arms onto different types of upper made of leather and/or of a thermoplastic polymer.

The said first ring 110 forms the first part of an articulation which also comprises:
- a second ring 120 from which a top-upper 20a arm 121 extends; the top-upper arm 121 also has a plurality of holes 121c which are uniformly spaced along its length so as to allow fixing of the arm to the said upper in/at different positions/heights of the latter.

The second ring 120 has at least two diametrically opposite eyelets 123 extending over a predefined annular section of the circumference of the ring and with a width such as to allow insertion, in the transverse direction Y-Y, of a respective pin 114 of the two which are joined to the first ring 110. The lengthwise extension of the eyelets 123 may be defined depending on the actual use of the footwear and/or the preferences of the user, as will emerge more clearly below.

A first annular body 130 provided with two diametrically opposite holes 134 with a diameter such as to allow the insertion of a respective pin 114 is arranged between the first ring 110 and the second ring 120 of the articulation; the annular body has two annular edges 131 and 132 situated opposite each other in the transverse direction Y-Y and with a diameter such as to allow insertion, with slight friction, of the rings 110 and 120, respectively, inside the central opening.

As illustrated, in the embodiment shown, the seats 113 of the first ring 110, the holes 134 of the intermediate body 130 and the eyelets 123 of the second ring 120 are aligned with each other along the transverse axis Y-Y so as to allow insertion of the pins 114; in addition, it is envisaged that the thickness of the annular body 130 and of the pins 114 may be such as to allow the pins 114 to pass through the first ring and the intermediate body for insertion of their free end inside the respective eyelet 123 of the second ring 120.

The articulation device 100 also comprises an outer cover 140 provided with two diametrically opposite holes 143 in turn aligned in the transverse direction Y-Y with the holes 134 and the seats 113 and with an inner diameter such as to allow the insertion of a respective screw 144 suitable for engagement with the female thread 114a of the respective pin 114.

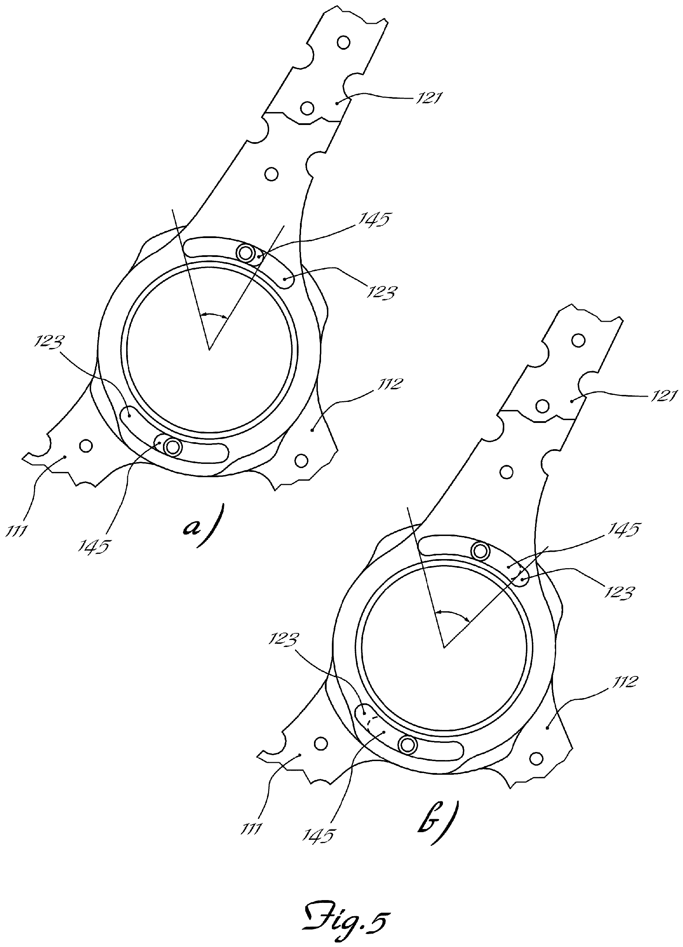

According to a preferred embodiment it is envisaged that, in addition to the holes 143 for receiving the screws 144, the cover 140 also has stops 145 projecting in the transverse direction (Y-Y) and arranged in suitable angular positions so as to be positioned between the pin 114 and the terminal end of the eyelet 123; the stop 145 therefore forms an end-of-travel stop (Fig. 5a,5b) for angular rotation of the second ring 120 and therefore of top-upper 20a the arm 121.

With such a configuration the operating principle of the footwear with articulation device is as follows:
-) depending on use of the footwear and the preferences of the user, the second ring 120 is prepared with eyelets 123 of suitable preselected length for determining the maximum possible working travel of the pins 114 inserted inside them and therefore the maximum rotational amplitude of the arm 121 of the top upper 20a with respect to the arm 111 of the bottom upper 20b joined to the insole 10;
-) the articulation device 100 is assembled by mounting, from the inside towards the outside in sequence in the transverse direction Y-Y, the following: the first ring 110 with bottom-upper 20b arms 111,112, the annular body 130, the second ring 120 with the top-upper 20a arm, and the pins 114 which are inserted inside the respective seat 113 and eyelet 123;
-) the arm 121 is applied to the top upper 20a;
-) preferably inserting the top-upper arm 121 inside a pocket of the top upper, thereby fastening the former to the latter;
-) the bottom-upper arms 111,112 are inserted inside special pockets in the said bottom upper, thereby fastening the former to the latter;
-) the cover 140 provided with stops 145 suitable for the intended use of the footwear is chosen and fitted;
-) the footwear is put on.

At this point the footwear is ready for use and is able to limit the respective plantar and dorsal rotations depending on the predefined length of the eyelets 123 of the second ring 120 and the prechosen cover, as well as the supination and pronation movements, owing to the rigidity provided by the fastening between the arms 111,112 and the bottom upper 20b and between the latter and the insole 10.

Preferably both the bottom-upper arms are inserted inside special seats formed in the upper and fixed by means of screws onto threaded dowel pins inserted inside them, so that the end of the front arm 111 is arranged in a position approximately corresponding to the metatarsal joint of the little toe and big toe of the user, while the rear arm is situated in the heel zone.

By providing different closing covers which are interchangeable with each other and have stops 145 arranged in different angular positions it is therefore possible to vary the degree of rotation of the upper, and therefore the foot, with respect to the insole by simply changing the said cover according to the use of the footwear and/or the individual preferences of the user, also where the footwear is provided ready-for-use with an external shell already applied to the upper.

For the specific case of use by a motorcyclist, for example, preferably three different angles of rotation of the articulated joint are provided, with an increasingly smaller degree of rotation for use in enduro, motocross or supercross racing, respectively.

For an application involving an orthopaedic shoe, the limitation of rotation of the articulated joint may, instead, be total with regard to the plantar and dorsal movements of the ankle.

According to the invention it is also envisaged that the particular ring-type configuration of the articulated joint 100 may allow the insertion of cushioning pads (not shown) which may be personalized, between the malleolus and the articulated joint in order to improve the comfort of the footwear.

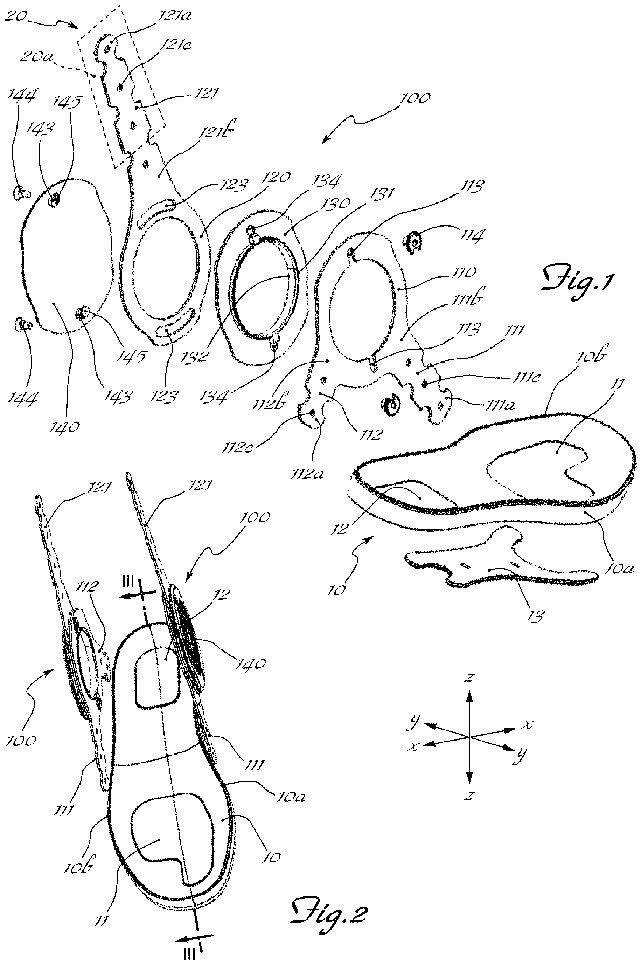

As shown in Fig. 1 in a preferred embodiment of the insole 10 of the footwear, said insole is hollowed out internally to a depth such as to allow better stitching together with the bottom upper, thus reducing the thickness of the shoe at the toe, and in particular is provided with two zones, i.e. a front zone 11 and rear zone 12, which are further hollowed out in the thickness of the insole so as to allow the insertion of soft material suitable for increasing the comfort during use and preventing irritation of the user's skin.

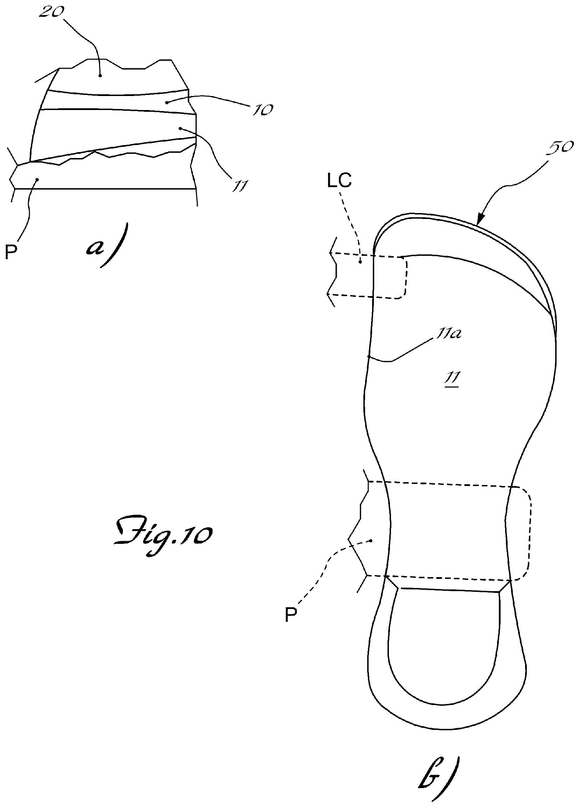

As shown in Fig. 10a, a sole 11 is applied to the insole 10, said sole 11 preferably having an asymmetrical form and greater height on the inner side and smaller height on the outer side in order to improve supporting on the footrest P of the motorcycle; in addition it is possible to design the insole 10 with an inner side 10a which is substantially straight; as shown in Fig.10b, the sole 11 is in turn preferably shaped with a straight inner side 11a for facilitating interaction with the gear lever LC shown in broken lines in the figure.

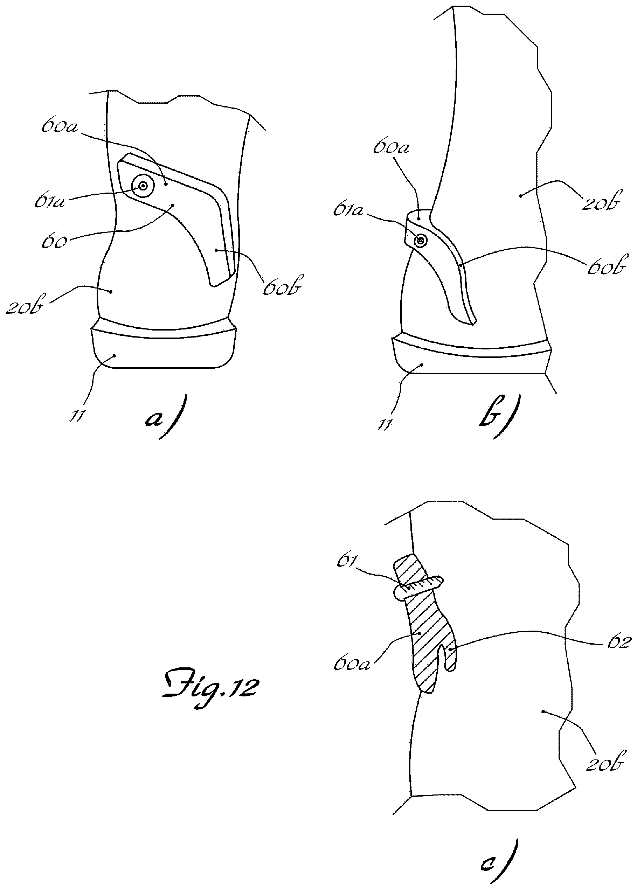

According to a further preferred embodiment shown in Fig. 12, the footwear according to the invention comprises an insert 60 which is applied to the bottom upper 20b in the vicinity of the heel and is designed to improve the interaction with the gear lever at the start of a race, when the foot is situated in front of the said gear lever, and facilitate changing from second gear to third gear without losing time, said gear changing operation being performed with the heel.

Conveniently, the insert 60 has a first substantially straight section 60a with a through-hole 61a and a second section 60b which is inclined from the top downwards and from the outside towards the inside.

The insert 60 is fixed to the upper 20b by means of a screw 61 inserted inside the through-hole 61a and a tooth 62 projecting from the surface of the inclined section 60b facing the upper 20b and suitable for insertion inside a heel-piece of the upper 20b inside which the tooth penetrates through a corresponding seat (not shown).

Fig. 1 also shows a stiffening plate 13 which can be fitted to the bottom surface of the insole in order to improve the rigidity of the insole in the foot-arch and ensure better absorption of impacts and greater stability whilst walking.

With the footwear according to the invention it is also possible to adjust the height of the articulated joint, which is not constrained to the insole, depending on the different positions of the inner and outer malleoli.

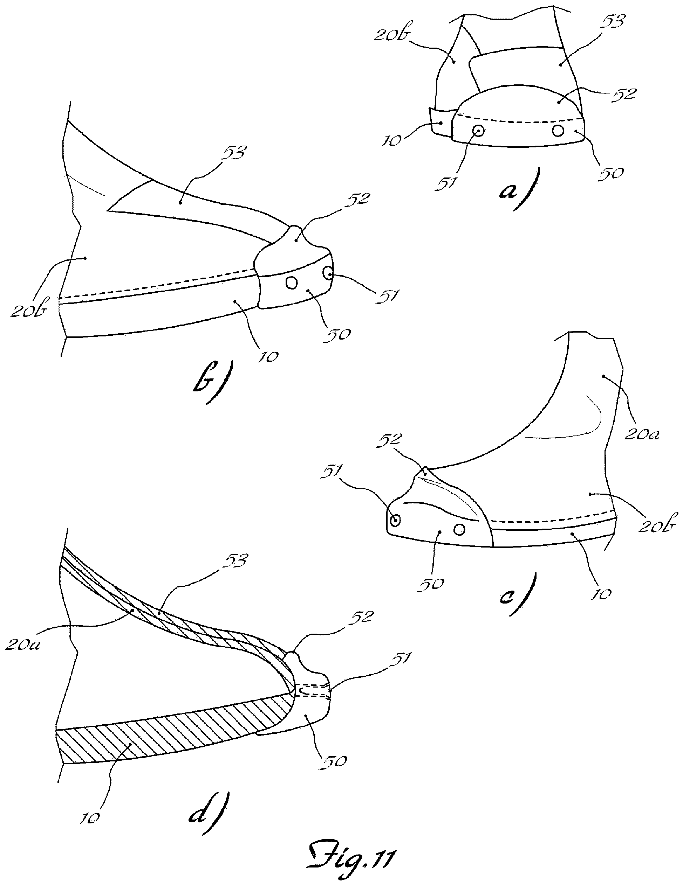

In addition, in a preferred embodiment of the footwear, shown in Fig. 11, it is envisaged that said footwear has a toe-piece 50 which is made of a plastic polymer and which is fixed, for example, by means of screws 51 and is suitably shaped with an upper raised profile 52 suitable for interaction with the gear lever (not shown) for more reliable and precise operation thereof. In the vicinity of the outer surface of the bottom upper 20b, the toe of the footwear also has a further upper reinforcement 53 designed to limit the wear arising from contact with the gear lever.

According to the invention it is envisaged that the thickness and the inner diameter of the rings 110,120 and the annular body 130 are preferably such as to allow retention of a shaped zone of the bottom upper, formed in the region of the malleolus, and of the malleolus itself, once the user has put on the footwear; this allows a significant reduction in the transverse volume of the footwear itself, in particular on its inner side, and therefore, for example in the case of motorcyclists' footwear, closer contact between the user's leg and the motorcycle frame and consequently a better hold thereon, this being particularly advantageous during use of the motorcycle for motorcycling trials, motocross and the like.

It is therefore clear how, with the footwear according to the invention, it is possible to achieve efficient and reliable control of the articulation device with positive and permanent definition of the possible relative rotation of foot/leg determined by the length of the eyelet in the second ring of the articulation device and by the configuration of the stops on the closing cover of the articulated joint.

In this connection it is envisaged that the footwear may be provided with a plurality of second rings having eyelets of different length, each of which specifically designed for the intended use and/or the individual requirements of the user.

In addition, of particular importance for the motorcycling sector is the fact that the design of the articulated joint with internally hollow rings allows a reduction in the thickness of the said articulated joint, this solution allowing the rider to grip more closely the motorcycle with the whole leg and not just the ankle part, as occurs with the footwear of the prior art which, having the articulated joint centred on the malleolus, result in a corresponding greater overall volume in the transverse direction.

The structure of the articulation device moreover, in the event of an impact, ensures a degree of dissipation of the impact energy transmitted to the foot and leg via the footrest P when landing after a jump or if the foot is in contact with the ground while travelling around a bend, which is much greater than that of the known footwear which necessarily concentrates this energy in the malleolus zone, thus ensuring better protection of the inner and outer malleolus region.

According to the invention it is also envisaged that the footwear may comprise movable means 200 for precise adaptation of the closure of the upper to the volume of the user's calf muscle.

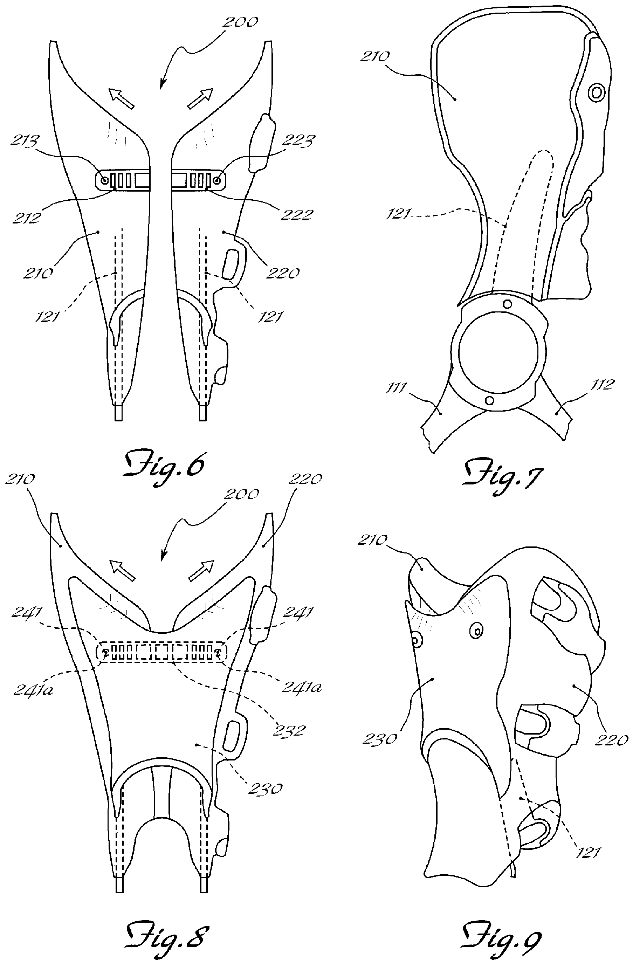

As shown in Figs. 6-9, these means 200 comprise substantially two flaps 210,220, i.e. right-hand flap and left-hand flap, which are designed to embrace the user's calf muscle; each flap 210,220 is fastened to the respective upper arm 121 of the articulated joint 100 and has on its rear a side rack section 212,222 designed to mesh with a corresponding rack 232 present on the front surface of a tongue 230 connecting together the two flaps 210,220; once adjustment has been performed, the flaps may be locked in position by means of screws 241 passing through holes 241a in the tongue 230 and screwed into a respective hole 212,213 with female thread in each flap 210,220.

Although described in connection with a number of embodiments and a number of preferred examples of embodiment of the invention, it is understood that the scope of protection of the present patent is determined solely by the claims below.

## Claims

1. Sports and/or orthopaedic footwear comprising an insole (10,10a,10b), an upper (20) formed by a top-upper part (20a) and a bottom-upper part (20b) joined to the insole (10), and an articulation device (100) for relative articulation of the insole/upper, said articulation device (100) comprising:
- a first ring (110) which has a bottom-upper front arm (111) and a bottom-upper rear arm (112) which are joined to said bottom-upper part (20b);
- a second ring (120) which has an arm (121) adapted to be fixed to the top-upper part (20a), said second ring (120) having at least two diametrically opposite eyelets (123) extending over a predefined annular section of the circumference of the second ring (120) itself;
- an anti-friction annular body (130) arranged between said first ring (110) and second ring (120) ;
- an outer cover (140) for closing the articulation device, provided with stops (145) each projecting outwards in the transverse direction (Y-Y) and designed to be inserted in a suitable angular position inside a respective one of the eyelets (123) of the second ring (120); wherein said first ring (110) has two diametrically opposite, radial, inner seats (113) for receiving a respective pin (114) with female thread (114a);
wherein the annular body (130) has two diametrically opposite holes (134) with a diameter such as to allow a respective one of said pins (114) to pass through,
wherein the outer cover (140) is provided with two diametrically opposite holes (143) in turn aligned in the transverse direction (Y-Y) with the holes (134) of the annular body (130) and the inner seats (113) of the first ring (110) and with an inner diameter such as to allow the insertion of a respective screw (144) suitable for engagement with the female thread (114a) of the respective pin (114);
and wherein, in use, the articulation device (100) is in a position substantially aligned with one of the malleoli of the user and includes it in the transverse direction within its thickness.

2. Footwear according to Claim 1 wherein said articulation device (100) is arranged on an inner side of the footwear in a position substantially aligned, in use, with the respective inner malleolus of the user,
**characterized in that** it further comprises a further articulation device (100) for relative articulation of the insole/upper, said further articulation device (100) arranged on the outer side of the footwear and comprising:
- a first ring which has a bottom-upper front arm and a bottom-upper rear arm which are joined to said bottom-upper part;
- a second ring which has an arm adapted to be fixed to the top-upper part, said second ring having at least two diametrically opposite eyelets extending over a predefined annular section of the circumference of the second ring itself;
- an anti-friction annular body arranged between said first ring and second ring;
- an outer cover for closing the articulation device, provided with stops each projecting outwards in the transverse direction and designed to be inserted in a suitable angular position inside a respective one of the eyelets of the second ring;
wherein said first ring has two diametrically opposite, radial, inner seats for receiving a respective pin with female thread;
wherein the annular body has two diametrically opposite holes with a diameter such as to allow a respective one of said pins to pass through, wherein the outer cover is provided with two diametrically opposite holes in turn aligned in the transverse direction (Y-Y) with the holes of the annular body and the inner seats of the first ring and with an inner diameter such as to allow the insertion of a respective screw suitable for engagement with the female thread of the respective pin;
and wherein, in use, the further articulation device (100) is in a position substantially aligned with the respective outer malleolus of the user and includes it in the transverse direction within its thickness.

3. Footwear according to any one of the preceding claims, **characterized in that** one of the two inner seats (113) of the first ring (110) is arranged between the two bottom-upper arms (111,112).

4. Footwear according to any one of the preceding claims, **characterized in that** the annular body (130) has two annular edges (131,132) projecting in opposite senses along the transverse direction (Y-Y) and with an outer diameter such as to allow the insertion, with slight friction inside the central opening, of the first ring (110) and the second ring (120), respectively.

5. Footwear according to any one of the preceding claims, **characterized in that** the seats (113) of the first ring (110), the holes (134) of the intermediate body (130) and the eyelets (123) of the second ring (120) of the articulation device (100) are, in use, aligned with each other along the transverse axis (Y-Y).

6. Footwear according to any one of the preceding claims, **characterized in that** the thickness of the annular body (130) and of the first ring (110) is such as to allow the pins (114) to pass through the first ring (110) and the annular body (130) for insertion of their free end inside the respective eyelet (123) of the second ring (120).

7. Footwear according to any one of the preceding claims, **characterized in that** the stops (145) projecting from the cover (140) in the transverse direction (Y-Y) are arranged in angular positions such as to be arranged between the pin (114) and a terminal end of the eyelet (123) of the second ring (120) so as to form a different end-of-travel stop for relative rotation of the first ring (110) and second ring (120).

8. Footwear according to any one of the preceding claims, **characterized in that** the front arm (111) is fixed to the bottom-upper part (20b) in a position approximately corresponding to the metatarsal joint of the little toe and big toe of the user.

9. Footwear according to any one of the preceding claims, **characterized in that** it comprises movable means (200) for precisely adapting the closure of the upper to the volume of the calf muscle of the user.

10. Footwear according to the preceding claim, **characterized in that** said means (200) for adapting the closure comprises two flaps (210,220) designed to grip the calf muscle of the user.

11. Footwear according to claims 10 and 2, wherein each flap (210,220) is fastened to the arm (121) of a respective articulation device (100) which is designed to be fixed to the top-upper part.

12. Footwear according to claim 10 or 11, **characterized in that** each flap (210,220) has on its rear face a rack section (212,222) which is designed to mesh with a corresponding rack (232) present on the front surface of a tongue (230) for connecting together the two flaps (210,220).

13. Footwear according to any one of the preceding claims, **characterized in that** a sole (11), preferably designed with an asymmetrical form having a greater height on the inner side and smaller height on the outer side, is applied to the insole (10).

14. Footwear according to any one of the preceding claims, **characterized in that** the insole (10) has an inner side (10a) which is substantially straight.

15. Footwear according to any one of the preceding claims, **characterized in that** a sole (11) which has a substantially straight inner side (11a) is applied to the insole (10).

16. Footwear according to any one of the preceding claims, **characterized in that** it has a reinforcing toe-piece (50) provided with concavity (51) applied to the toe of the said footwear.

17. Footwear according to any one of the preceding claims, **characterized in that** it has a reinforcement (53) applied to the bottom-upper part (20b) in the vicinity of the outer surface of a toe of the footwear.

18. Footwear according to any one of the preceding claims, **characterized in that** it comprises an insert (60) applied to the bottom-upper part (20b) in the vicinity of a heel of the footwear.

19. Footwear according to the preceding claim, **characterized in that** the insert (60) has a first section (60a) which is substantially straight and a second section (60b) which is inclined from the top downwards and from the outside towards the inside.

20. Footwear according to Claim 19, **characterized in that** the insert (60) has a through-hole (61a) for a screw (61) for fixing to the upper and a tooth (62) projecting from the surface of the inclined section (60b) facing the upper (20b) and designed to be inserted inside a corresponding seat in a heel-piece of the said upper.

## Patentansprüche

1. Sport- und/oder orthopädisches Schuhwerk, umfassend eine Innensohle (10, 10a, 10b), einen Schaft (20), der durch ein Oberschaftteil (20a) und ein Unterschaftteil (20b) gebildet ist, das mit der Innensohle (10) verbunden ist, und eine Gelenkvorrichtung (100) zur relativen Artikulation von Innensohle/Schaft, wobei die Gelenkvorrichtung (100) umfasst:
- einen ersten Ring (110), der einen Unterschaft-Vorderarm (111) und Unterschaft-Hinterarm (112) aufweist, die mit dem genannten Unterschaftteil (20b) verbunden sind;
- einen zweiten Ring (120), der einen Arm (121) aufweist, der angepasst ist, an dem Oberschaftteil (20a) befestigt zu werden, wobei der zweite Ring (120) mindestens zwei diametral gegenüberliegende Öffnungen (123) aufweist, die sich ihrerseits über einen vorbestimmten ringförmigen Abschnitt des Umfangs des zweiten Rings (120) erstrecken;
- einen reibungsarmen ringförmigen Körper (130), der zwischen dem ersten Ring (110) und dem zweiten Ring (120) angeordnet ist;
- eine äußere Abdeckung (140) zum Schließen der Gelenkvorrichtung, die mit Anschlägen (145) versehen ist, die jeweils in Querrichtung (Y-Y) nach außen ragen und dazu bestimmt sind, in einer geeigneten Winkellage in eine entsprechende der Öffnungen (123) des zweiten Rings (120) eingesetzt zu werden; wobei der erste Ring (110) zwei diametral gegenüberliegende, radiale Innensitze (113) zur Aufnahme eines jeweiligen Stiftes (114) mit Innengewinde (114a) aufweist;
wobei der ringförmige Körper (130) zwei diametral gegenüberliegende Löcher (134) mit einem Durchmesser aufweist, der es ermöglicht, dass ein entsprechender der Stifte (114) durchgeführt wird;
wobei die äußere Abdeckung (140) mit zwei diametral gegenüberliegenden Löchern (143) versehen ist, die wiederum in Querrichtung (Y-Y) zu den Löchern (134) des ringförmigen Körpers (130) und den Innensitzen (113) des ersten Rings (110) ausgerichtet sind, und mit einem Innendurchmesser, der das Einsetzen einer entsprechenden Schraube (144) ermöglicht, die zum Eingriff mit dem Innengewinde (114a) des jeweiligen Stiftes (114) geeignet ist;
und wobei die Gelenkvorrichtung (100) im Gebrauch in einer Position ist, die im Wesentlichen zu einem der Malleolen des Benutzers ausgerichtet ist und ihn in Querrichtung innerhalb seiner Dicke einschließt.

2. Schuhwerk nach Anspruch 1, wobei die Gelenkvorrichtung (100) auf einer Innenseite des Schuhwerks in einer Position angeordnet ist, die im Gebrauch im Wesentlichen zu dem jeweiligen inneren Knöchel des Benutzers ausgerichtet ist,
**dadurch gekennzeichnet, dass** es ferner eine weitere Gelenkvorrichtung (100) zur relativen Artikulation von Innensohle/Schaft umfasst, wobei die weitere Gelenkvorrichtung (100) auf der Außenseite des Schuhwerks angeordnet ist und umfasst:
- einen ersten Ring, der einen Unterschaft-Vorderarm und einen Unterschaft-Hinterarm aufweist, die mit dem genannten Unterschaftteil verbunden sind;
- einen zweiten Ring, der einen Arm aufweist, der angepasst ist, an dem Oberschaftteil befestigt zu werden, wobei der zweite Ring mindestens zwei diametral gegenüberliegende Öffnungen aufweist, die sich über einen vorbestimmten ringförmigen Abschnitt des Umfangs des zweiten Rings selbst erstrecken;
- einen reibungsarmen ringförmigen Körper, der zwischen dem ersten Ring und dem zweiten Ring angeordnet ist;
- eine äußere Abdeckung zum Schließen der Gelenkvorrichtung, die mit Anschlägen versehen ist, die jeweils in Querrichtung nach außen ragen und dazu bestimmt sind, in einer geeigneten Winkellage in eine entsprechende der Öffnungen des zweiten Rings eingesetzt zu werden;
wobei der erste Ring zwei diametral gegenüberliegende, radiale Innensitze zur Aufnahme eines jeweiligen Stiftes mit Innengewinde aufweist;
wobei der ringförmige Körper zwei diametral gegenüberliegende Löcher mit einem Durchmesser aufweist, der es ermöglicht, dass ein entsprecher der Stifte durchgeführt wird,
wobei die äußere Abdeckung mit zwei diametral gegenüberliegenden Löchern versehen ist, die wiederum in Querrichtung (Y-Y) zu den Löchern des ringförmigen Körpers und den Innensitzen des ersten Rings ausgerichtet sind, und mit einem Innendurchmesser, der das Einsetzen einer entsprechenden Schraube ermöglicht, die zum Eingriff mit dem Innengewinde des jeweiligen Stiftes geeignet ist;
und wobei im Gebrauch die weitere Gelenkvorrichtung (100) in einer Position ist, die im Wesentlichen zu dem jeweiligen äußeren Knöchel des Benutzers ausgerichtet ist und ihn in Querrichtung innerhalb seiner Dicke einschließt.

3. Schuhwerk nach einem der vorstehenden Ansprüche, **dadurch gekennzeichnet, dass** einer der beiden Innensitze (113) des ersten Rings (110) zwischen den beiden Unterschaft-Armen (111, 112) angeordnet ist.

4. Schuhwerk nach einem der vorstehenden Ansprüche, **dadurch gekennzeichnet, dass** der ringförmige Körper (130) zwei ringförmige Kanten (131, 132) aufweist, die in entgegengesetzter Richtung entlang der Querrichtung (Y-Y) vorstehen und einen Außendurchmesser besitzen, der das Einführen des ersten Rings (110) bzw. des zweiten Rings (120) mit geringer Reibung innerhalb der zentralen Öffnung ermöglicht.

5. Schuhwerk nach einem der vorstehenden Ansprüche, **dadurch gekennzeichnet, dass** die Sitze (113) des ersten Rings (110), die Löcher (134) des Zwischenkörpers (130) und die Öffnungen (123) des zweiten Rings (120) der Gelenkvorrichtung (100) im Gebrauch entlang der Querachse (Y-Y) zueinander ausgerichtet sind.

6. Schuhwerk nach einem der vorstehenden Ansprüche, **dadurch gekennzeichnet, dass** die Dicke des ringförmigen Körpers (130) und des ersten Rings (110) so bemessen ist, dass die Stifte (114) den ersten Ring (110) und den ringförmigen Körper (130) durchdringen können, um ihr freies Ende in die jeweilige Öffnung (123) des zweiten Rings (120) einzuführen.

7. Schuhwerk nach einem der vorhergehenden Ansprüche, **dadurch gekennzeichnet, dass** die Anschläge (145), die von der Abdeckung (140) in Querrichtung (Y-Y) vorstehen, in Winkellagen so angeordnet sind, dass sie zwischen dem Stift (114) und einem Ende der Öffnung (123) des zweiten Rings (120) angeordnet sind, um einen anderen Endanschlag für die relative Drehung des ersten Rings (110) und des zweiten Rings (120) zu bilden.

8. Schuhwerk nach einem der vorstehenden Ansprüche, **dadurch gekennzeichnet, dass** der Vorderarm (111) am Unterschaftteil (20b) in einer Position befestigt ist, die etwa dem Mittelfußgelenk der kleinen Zehe und der großen Zehe des Benutzers entspricht.

9. Schuhwerk nach einem der vorstehenden Ansprüche, **dadurch gekennzeichnet, dass** es bewegliche Mittel (200) umfasst, um den Verschluss des Schafts genau an das Volumen des Wadenmuskels des Benutzers anzupassen.

10. Schuhwerk nach dem vorstehenden Anspruch, **dadurch gekennzeichnet, dass** die Mittel (200) zur Anpassung des Verschlusses zwei Klappen (210, 220) umfassen, die dazu bestimmt sind, den Wadenmuskel des Benutzers zu greifen.

11. Schuhwerk nach den Ansprüchen 10 und 2, wobei jede Klappe (210, 220) am Arm (121) einer jeweiligen Gelenkvorrichtung (100) befestigt ist, die zur Befestigung am Oberschaftteil bestimmt ist.

12. Schuhe nach Anspruch 10 oder 11, **dadurch gekennzeichnet, dass** jede Klappe (210, 220) auf ihrer Rückseite einen Zahnstangenabschnitt (212, 222) aufweist, der dazu bestimmt ist, mit einem entsprechenden Zahnstangenabschnitt (232), der auf der Vorderseite einer Zunge (230) zum Verbinden der beiden Klappen (210, 220) vorhanden ist, in Eingriff zu kommen.

13. Schuhwerk nach einem der vorstehenden Ansprüche, **dadurch gekennzeichnet, dass** eine Sohle (11), die vorzugsweise mit einer asymmetrischen Form mit einer größeren Höhe auf der Innenseite und einer kleineren Höhe auf der Außenseite ausgebildet ist, auf die Innensohle (10) aufgebracht ist.

14. Schuhwerk nach einem der vorhergehenden Ansprüche, **dadurch gekennzeichnet, dass** die Innensohle (10) eine Innenseite (10a) aufweist, die im Wesentlichen gerade ist.

15. Schuhwerk nach einem der vorstehenden Ansprüche, **dadurch gekennzeichnet, dass** auf die Innensohle (10) eine Sohle (11) aufgebracht ist, die eine im Wesentlichen gerade Innenseite (11a) aufweist.

16. Schuhwerk nach einem der vorstehenden Ansprüche, **dadurch gekennzeichnet, dass** es ein Verstärkungszehenstück (50) aufweist, das mit einer Vertiefung (51) versehen ist, die auf die Spitze des Schuhwerks aufgebracht ist.

17. Schuhwerk nach einem der vorstehenden Ansprüche, **dadurch gekennzeichnet, dass** es eine Verstärkung (53) aufweist, die auf das Unterschaftteil (20b) in der Nähe der Außenfläche einer Zehe des Schuhwerks aufgebracht ist.

18. Schuhwerk nach einem der vorstehenden Ansprüche, **dadurch gekennzeichnet, dass** es einen Einsatz (60) umfasst, der auf das Unterschaftteil (20b) in der Nähe einer Ferse des Schuhwerks aufgebracht ist.

19. Schuhwerk nach dem vorstehenden Anspruch, **dadurch gekennzeichnet, dass** der Einsatz (60) einen ersten Abschnitt (60a), der im Wesentlichen gerade ist, und einen zweiten Abschnitt (60b), der von oben nach unten und von außen nach innen geneigt ist, aufweist.

20. Schuhwerk nach Anspruch 19, **dadurch gekennzeichnet, dass** der Einsatz (60) ein Durchgangsloch (61a) für eine Schraube (61) zur Befestigung am Schaft und einen Zahn (62) aufweist, der von der dem Schaft (20b) zugewandten Oberfläche des geneigten Abschnitts (60b) vorsteht und dazu bestimmt ist, in einen entsprechenden Sitz in einem Fersenteil des Schafts eingesetzt zu werden.

## Revendications

1. Chaussure de sport et/ou orthopédique comprenant une semelle intérieure (10, 10a, 10b), une empeigne (20) formée par une partie empeigne supérieure (20a) et une partie empeigne inférieure (20b) reliées à la semelle intérieure (10), et un dispositif d'articulation (100) destiné à une articulation relative de la semelle intérieure/empeigne, ledit dispositif d'articulation (100) comprenant :
- un premier anneau (110) qui possède un bras avant (111) d'empeigne inférieure et un bras arrière (112) d'empeigne inférieure qui sont reliés à ladite partie empeigne inférieure (20b),
- un second anneau (120) qui possède un bras (121) conçu pour être fixé à la partie empeigne supérieure (20a), ledit second anneau possédant au moins deux œillets (123) diamétralement opposés s'étendant sur une section annulaire prédéfinie de la circonférence du second anneau (120) lui-même,
- un corps annulaire anti-frottement (130) agencé entre ledit premier anneau (110) et ledit second anneau (120),
- une protection extérieure (140) destinée à fermer le dispositif d'articulation, dotée de butées (145), chacune faisant saillie vers l'extérieur dans la direction transversale (Y-Y) et conçues pour être introduites dans des positions angulaires appropriées à l'intérieur de l'un, respectif, des œillets (123) du second anneau (120),
dans laquelle ledit premier anneau (110) possède deux surfaces d'appui (113) intérieures radiales diamétralement opposées destinées à recevoir une cheville (114) respective avec un filetage femelle (114a),
dans laquelle le corps annulaire (130) possède deux trous (134) diamétralement opposés ayant un diamètre tel qu'il permet à l'une, respective, desdites chevilles (114), de passer à travers,
dans laquelle la protection extérieure (140) est dotée de deux trous (143) diamétralement opposés à leur tour alignés dans la direction transversale (Y-Y) avec les trous (1334) du corps annulaire (130) et les surfaces d'appui (113) intérieures du premier anneau (110) et ayant un diamètre intérieur tel qu'il permet l'introduction d'une vis (144) respective appropriée pour un engagement sur le filetage femelle (114a) de la cheville (114) respective
et dans laquelle, en cours d'utilisation, le dispositif d'articulation (100) est dans une position sensiblement alignée avec l'une des malléoles de l'utilisateur et l'inclut dans la direction transversale dans son épaisseur.

2. Chaussure selon la revendication 1, dans laquelle ledit dispositif d'articulation (100) est agencé sur un côté intérieur de la chaussure dans une position sensiblement alignée, pendant l'utilisation, avec la malléole interne respective de l'utilisateur,
**caractérisée en ce qu'**elle comprend en outre un autre dispositif d'articulation (100) assurant une articulation relative de la semelle intérieure/empeigne, ledit autre dispositif d'articulation (100) étant agencé sur le côté extérieur de la chaussure et comprenant :
- un premier anneau qui possède un bras avant d'empeigne inférieure et un bras arrière d'empeigne inférieure qui sont réunis à ladite partie empeigne supérieure,
- un second anneau qui possède un bras conçu pour être fixé sur la partie empeigne supérieure, ledit second anneau ayant au moins deux œillets diamétralement opposés qui s'étendent sur une section annulaire prédéfinie de la circonférence du second anneau lui-même,
- un corps annulaire anti-frottement agencé entre lesdits premier anneau et second anneau,
- une protection extérieure destinée à fermer le dispositif d'articulation, dotée de butées, faisant chacune saillie vers l'extérieur, dans la direction transversale et conçues pour être introduites, dans une position angulaire appropriée, à l'intérieur de l'un, respectif, des œillets du second anneau,
dans laquelle ledit premier anneau possède deux surfaces d'appui intérieures radiales diamétralement opposées pour recevoir une cheville respective dotée d'un filetage femelle,
dans laquelle le corps annulaire possède deux trous diamétralement opposés ayant un diamètre tel qu'il permet que l'une, respective, desdites chevilles passe à travers,
dans laquelle la protection extérieure est dotée de deux trous diamétralement opposés à leur tour alignés dans la direction transversale (Y-Y) avec les trous du corps annulaire et les surfaces d'appui intérieures du premier anneau et ayant un diamètre intérieur tel qu'il permet l'insertion d'une vis respective adaptée pour s'engager sur le filetage femelle de la cheville respective
et dans laquelle, en cours d'utilisation, l'autre dispositif d'articulation (100) est dans une position sensiblement alignée avec la malléole externe respective de l'utilisateur et l'inclut dans la direction transversale dans son épaisseur.

3. Chaussure selon l'une quelconque des revendications précédentes, **caractérisée en ce que** l'une des deux surfaces d'appui intérieures (113) du premier anneau (110) est agencée entre les deux bras d'empeigne inférieure (111, 112).

4. Chaussure selon l'une quelconque des revendications précédentes, **caractérisée en ce que** le corps annulaire (130) possède deux bords annulaires (131, 132) qui font saillie dans des sens opposés dans la direction transversale (Y-Y) et ayant un diamètre extérieur tel qu'il permet l'insertion, avec un léger frottement, à l'intérieur de l'ouverture centrale, du premier anneau (110) et du second anneau (120), respectivement.

5. Chaussure selon l'une quelconque des revendications précédentes, **caractérisée en ce que** les surfaces d'appui (113) du premier anneau (110), les trous (134) du corps intermédiaire (130) et les œillets (123) du second anneau (120) du dispositif d'articulation (100) sont, pendant l'utilisation, alignés les uns avec les autres le long de l'axe transversal (Y-Y).

6. Chaussure selon l'une quelconque des revendications précédentes, **caractérisée en ce que** l'épaisseur du corps annulaire (130) et du premier anneau (110) est telle qu'elle permet aux chevilles (114) de passer à travers le premier anneau (110) et le corps annulaire (130) aux fins d'insertion de leur extrémité libre à l'intérieur de l'œillet (123) respectif du second anneau (120).

7. Chaussure selon l'une quelconque des revendications précédentes, **caractérisée en ce que** les butées (145) qui font saillie à partir de la protection (140) dans la direction transversale (Y-Y) sont agencées dans des positions angulaires de telle sorte qu'elles sont agencées entre la cheville (114) et une extrémité terminale de l'œillet (123) du second anneau (120) de manière à former une butée de fin de course différente pour une rotation relative du premier anneau (110) et du second anneau (120).

8. Chaussure selon l'une quelconque des revendications précédentes, **caractérisée en ce que** le bras avant (111) est fixé à la partie empeigne inférieure (20b) dans une position qui correspond approximativement à l'articulation métatarsienne du petit orteil et du gros orteil de l'utilisateur.

9. Chaussure selon l'une quelconque des revendications précédentes, **caractérisée en ce qu'**elle comprend des moyens mobiles (200) permettant d'adapter avec précision la fermeture de l'empeigne au volume du muscle du mollet de l'utilisateur.

10. Chaussure selon les revendications précédentes, **caractérisée en ce que** ledit moyen (200) d'adaptation de la fermeture comprend deux rabats (210, 220) conçus pour enserrer le muscle du mollet de l'utilisateur.

11. Chaussure selon les revendications 10 et 2, dans laquelle chaque rabat (210, 220) est fixé sur le bras (121) d'un dispositif d'articulation (100) respectif qui est conçu pour être fixé sur la partie empeigne supérieure.

12. Chaussure selon la revendication 10 ou 11, **caractérisée en ce que** chaque rabat (210, 220) possède, sur sa face arrière, une section de support (212, 222) qui est conçue pour s'engager sur un support (232) correspondant présent sur la surface avant d'une languette (230) pour relier ensemble les deux rabats (210, 220).

13. Chaussure selon l'une quelconque des revendications précédentes, **caractérisée en ce qu'**une semelle (11), de préférence conçu avec une forme asymétrique, ayant une hauteur plus grande sur le côté intérieur et une hauteur plus petite sur le côté extérieur, est appliquée sur la semelle intérieure (10) .

14. Chaussure selon l'une quelconque des revendications précédentes, **caractérisée en ce que** la semelle intérieure (10) possède un côté intérieur (10a) qui est sensiblement droit.

15. Chaussure selon l'une quelconque des revendications précédentes, **caractérisée en ce qu'**une semelle (11) qui possède un côté interne sensiblement droit (11a) est appliquée sur la semelle intérieure (10) .

16. Chaussure selon l'une quelconque des revendications précédentes, **caractérisée en ce qu'**elle possède un élément de bout de renforcement (50) ayant une concavité (51) appliquée sur le bout de ladite chaussure.

17. Chaussure selon l'une quelconque des revendications précédentes, **caractérisée en ce qu'**elle possède un renfort (53) appliqué sur la partie empeigne inférieure (20b) à proximité de la surface extérieure d'un bout de la chaussure.

18. Chaussure selon l'une quelconque des revendications précédentes, **caractérisée en ce qu'**elle comprend un entre-deux (60) appliqué à la partie empeigne inférieure (20b) à proximité d'un talon de la chaussure.

19. Chaussure selon la revendication précédente, **caractérisée en ce que** l'entre-deux (60) possède une première section (60a) qui est sensiblement droite et une seconde section (60b) qui est inclinée depuis le haut vers le bas, et depuis l'extérieur vers l'intérieur.

20. Chaussure selon la revendication 19, **caractérisée en ce que** l'entre-deux (60) possède un trou traversant (61a) pour une vis (61) afin d'être fixé à l'empeigne et une dent (62) qui fait saillie à partir de la surface de la section inclinée (60b) qui fait face à l'empeigne (20b) et conçue pour être introduite à l'intérieur d'une surface d'appui correspondant dans une talonnette de ladite empeigne.
